# EUROPEAN PATENT SPECIFICATION

(11) **EP 2 416 708 B1**
(45) Date of publication and mention of the grant of the patent: **08.05.2019**
(21) Application number: 09788174.2
(22) Date of filing: 01.04.2009
(51) Int. Cl.: A61B 5/103, G01N 21/65, C12Q 1/68

(54) **METHOD FOR DETERMINATION OF ONE OR MORE MUTATIONS**
VERFAHREN ZUR BESTIMMUNG VON EIN ODER MEHR MUTATIONEN
PROCÉDÉ DE DÉTERMINATION D'UNE OU PLUSIEURS MUTATIONS

(43) Date of publication of application: 15.02.2012
(73) Proprietor: Erasmus University Medical Center Rotterdam, 3015 GE Rotterdam (NL)
(72) Inventor: CASPERS, Peter Jacobus, 3015 GE Rotterdam (NL); O'REGAN, Grainne, Dublin 12 (IE); DE STERKE, Johanna, 3029 AK Rotterdam (NL); PUPPELS, Gerwin Jan, 3015 GE Rotterdam (NL)
(74) Representative: V.O.
(86) International application number: PCT/NL2009/050166
(87) International publication number: WO 2010/114361

(56) References cited:
- WO-A-2009/002149
- WO-A-2009/002170
- WO-A1-2009/002170
- WO-A1-2009/002170
- WO-A2-2008/052221
- WO-A2-2008/052221
- US-A1- 2003 124 553
- CHRIT L ET AL: "In vivo chemical investigation of human skin using a confocal Raman fiber optic microprobe" JOURNAL OF BIOMEDICAL OPTICS, SPIE, PO BOX 10 BELLINGHAM WA 98227-0010 USA, vol. 10, no. 4, 1 July 2005 (2005-07-01), pages 44007-1, XP002471926 ISSN: 1083-3668
- KEMPERMAN P ET AL: "Abstracts for the International Investigative Dermatology 2008; Loss-of-function mutations in the filaggrin gene predicted by in vivo Raman spectrsocopy" JOURNAL OF DERMATOLOGICAL SCIENCE, ELSEVIER SCIENCE PUBLISHERS, SHANNON, IR, vol. 50, no. 2, 1 April 2008 (2008-04-01), pages E104-E104, XP002547072 ISSN: 0923-1811

## Description

### FIELD OF THE INVENTION

The invention is directed to a method for non-invasive determination if a person has one or two loss-of-function mutation(s) in the gene encoding for filaggrin.

### BACKGROUND OF THE INVENTION

Atopic dermatitis (AD) is a major problem in dermatology. Estimates for the prevalence of AD in developed countries range between 15 % and 20 %. [Roll et al. Curr. Opin. Allergy Clin. Immunol. 2004, 4(5), 373-378] AD represents an enormous burden on health care in general.

The outermost layer of the skin, the stratum corneum, is the main protective barrier of the body against water loss and penetration of harmful agents. An impaired barrier function is likely to be a primary event in AD.

AD is a common chronic inflammatory skin disease characterized by itchy, inflamed skin. [Roll et al. Curr. Opin. Allergy Clin. Immunol. 2004, 4(5), 373-378 and Stemmler et al. J. Invest. Dermatol. 2007, 127, 722-724] It is also well known that a predisposition for AD impairs the ability of a person to be active in certain professions such as hair-dressing and cookery.

Current opinion is that early detection of the predisposition of young children to develop AD and targeted treatment of the skin barrier can prevent further development of the atopic syndrome.

The protein filaggrin is of crucial importance for the formation and maintenance of the skin barrier. The protein is necessary in giving the correct macrostructure to the keratin cytoskeleton and it provides the amino acids for the production of the natural moisturising factor (NMF).

FLG mutations are the strongest and most widely replicated genetic risks for eczema identified to date [O'Regan et al. J Allergy Clin Immunol 2008, 122, 689-693]. Partial or complete loss of the ability to produce filaggrin results in an impaired barrier function and diminished epidermal defence mechanisms to allergens and microbes, which may result in eczema and chronic inflammation, including atopic dermatitis, AD-related asthma and allergies. These are major and increasing problems in the developed nations. [Sandilands et al. J. Invest. Dermatol. 2006, 126, 1770-1775; Sandilands et al. Nature Genetics 2006, 38, 337-342; Smith et al. Nature Genetics 2007, 39, 650-654; and Irvine et al. J. Invest. Dermatol. 2006, 126, 1200-1202]

Approximately 10 % of the people of European origin carry a mutation in the filaggrin gene. [Sandilands et al. J. Invest. Dermatol. 2006, 126, 1770-1775]. Carriers of a mutation can be heterozygous, which means that a mutation is present on only one of the two alleles. Carriers of a mutation can also be homozygous, which means that the mutation is present in both alleles. Carriers of a mutation can also be compound heterozygous, which means that both alleles contain a different mutation. Current opinion is that carriers of two filaggrin mutations have a stronger predisposition for AD than carriers of one mutation. To date a total of 37 loss-of-function mutations in the filaggrin gene have been identified [O'Regan et al. J Allergy Clin Immunol 2008, 122, 689-693].

Atopic dermatitis has different pathogenic features and a heterogeneous phenotype [Elias et al. J Allergy Clin Immunol 2008, 121, 1337]. This creates a strong rationale for deployment of specific therapeutic strategies and a demand for a new classification of the heterogeneous disease. Identification of FLG mutations may lead to a new, molecular classification of eczema, which may be the basis for targeted intervention and therapy of eczema.

Known methods to determine mutations in the filaggrin gene use specialised genotyping techniques, see for instance US-A-2003/0 124 553. Analysis of 15 variants of the filaggrin mutation is described by Sandilands et al. in Nature Genetics 2007, 39, 650-654. These genotyping methods are expensive and time-consuming and require highly specialised laboratory facilities and personnel. Moreover, costs, time and efforts increase with the number of variants of the filaggrin mutations screened. As a consequence, analysis for filaggrin mutations is generally limited to a subset of all known possible mutations, with the risk of missing less prevalent and previously unknown filaggrin mutations. Genotyping methods are unsuitable for population screening purposes.

Accordingly, a need exists for a quick and easy-to-use method to screen the population for the potential presence of one or two loss-of-function mutations in the filaggrin gene. In WO 2009/002149 and WO2009/002170 it has already been described how in general mutations in the filaggrin gene can be detected.

However, there is a need for a quick and easy-to-use method to determine whether an atopic dermatitis patient has one or two loss-of-function mutations in the filaggrin gene (i.e. whether the patients possesses a homozygous mutation or a compound heterozygous mutation) for a correct diagnosis and classification of the disease and choice of appropriate treatment. Such a diagnosis will increase the possibility of subgrouping the otherwise heterogenic disease of atopic dermatitis and will thereby enable a better phenotype-genotype characterization. This could improve preventive initiatives, secure better information of patients about the prognosis for their disease, and possible enable targeted treatment [C. Giwercman et al, Contact Dermatitis 2008, 59:257-260]

### SUMMARY OF THE INVENTION

The invention is defined in the appended claims and comprises a method to determine if a person has a homozygous or compound heterozygous mutation in the filaggrin-gene comprising measurement of the presence of tyrosine in the skin. Preferably in such a method Raman spectroscopy is used to obtain a measure of tyrosine presence in the skin and more preferably in the stratum corneum of the skin. In such a case, preferably the Raman measurements are taken of the palm of the hand.

In another embodiment of the invention multiple Raman measurements are carried out at more than one location of the body, preferably at more than one location of the hand. Further, the invention comprises a method according to the invention to determine if a person has a mutation in both FLG alleles, comprising measuring Raman spectra at different locations in the stratum corneum, determining if the tyrosine content is above a set threshold, whereby said person is likely to have a mutation in both FLG alleles or determining if the tyrosine content is below a set threshold, whereby said person is not likely to have a mutation in both FLG alleles.

The invention also comprises a method to determine if a person has zero, one or two mutant FLG-genes comprising measurement of the presence of tyrosine and the concentration of NMFin the skin, preferably by taking Raman spectra measurements at different locations in the stratum corneum, determining the NMF concentration and the presence tyrosine from the measured Raman spectra, determining that if the average NMF concentration is above a set threshold the said person is likely to have no FLG mutation, and determining that if the average NMF concentration is below a set threshold and the tyrosine content in all measurements is below a set threshold said person is likely to have a mutation in one FLG allele, and determining that if the average NMF concentration is below a set threshold and the tyrosine content in one or more measurements is above a set threshold said person is likely to have a mutation in both FLG alleles.

In one embodiment the measurement of tyrosine in a method according to the invention is performed *in vitro.*

Further part of the invention is a method according to the invention claims to test subjects for suitability of inclusion in a test of a topically applied product. Alternatively, a method according to the present invention can be used for determining whether an individual is unsuitable, or at least less suitable, for a certain profession or activity.

In another embodiment, the invention comprises a method to classify atopic dermatitis based on the NMF and tyrosine content in the skin of a subject.

In an example, not being part of the invention, a method to determine the presence of an unknown mutation in a gene is described, comprising measuring a first Raman spectrum of a first tissue without a mutation in said gene, measuring a second Raman spectrum of a corresponding second tissue with a known gene mutation in said gene, measuring a third Raman spectrum of a corresponding third tissue without a known mutation in said gene, and determining if said third Raman spectrum shows features similar to said second Raman spectrum which differ from said first Raman spectrum, and, if so, concluding that said gene in tissue has a mutation that is different from said known gene mutation. Preferably in such a method the tissue is skin and also preferably the gene is the filaggrin gene

Thus, it is an object of the present invention to provide for a method and apparatus for rapid non-invasive determination of the likelihood of the presence of two loss-of-function mutations in the filaggrin gene in a subject, particularly a human subject.

It is an object of the invention, combined with the method as described in WO 2009/002149 to provide for a method and apparatus for rapid non-invasive determination of the likelihood of the presence of one or two loss-of-function mutations in the filaggrin gene in a subject, particularly a human subject.

It is a further object of the invention to provide for a rapid and objective method to distinguish between a single mutation (heterozygous) or two mutations (homozygous or compound heterozygous) in the filaggrin gene.

It is a further object of this invention to provide for a method to determine the presence of one or more previously unknown mutations in a gene.

It is a further object of the invention to provide for a rapid and objective method to constitute suitable panels of individuals for studies concerning determination of penetration and/or effects of topically applied products, *i*.*e*. to provide objective measures to include or exclude individuals from study groups.

It is a further object of the invention to provide for an objective method to determine whether an individual is unsuitable, or at least less suitable, for a certain profession or activity because of an increased risk to develop an occupational skin problem.

It is a further object of the invention to provide for an objective method facilitating population screening to determine whether a young child has a predisposition for skin conditions related to one or more loss-of-function mutation(s) in the filaggrin gene, and thereby enable early intervention with preventive treatment and/or direct the patient to further diagnostic tests.

It is a further object of this invention to provide for an objective method to determine whether an individual suffering from atopic dermatitis has a filaggrin defect and to adjust the therapy accordingly.

It is a further object of this invention to provide for an objective method to classify the filaggrin defect in an individual suffering from atopic dermatitis and to adjust therapy accordingly.

### BRIEF DESCRIPTION OF THE DRAWINGS

Figure 1 is an example of an *in vivo* Raman spectrum of the stratum corneum on the palm of the hand of a subject with no filaggrin mutations.
Figure 2 is an example of an *in vivo* Raman spectrum of the stratum corneum on the palm of the hand of a subject with two filaggrin mutations from a region with elevated Tyr.
Figure 3 is an example of an *in vivo* Raman spectrum of the stratum corneum on the palm of the hand of a subject with two filaggrin mutations from a region with strongly elevated Tyr.
Figure 4 is an example of a Raman spectrum of solid L-tyrosine.
Figure 5 shows a histogram of the Tyr content for all volunteers included in the study of the example. The vertical axis is plotted compressed for clearer presentation.
Figure 6. Magnification of figure 5, showing that the Tyr content approximates a binomial distribution around a value defined as the normal Tyr content in normal skin.

### DETAILED DESCRIPTION OF THE INVENTION

The term "vibrational spectroscopy" as used herein is defined as any spectroscopic technique that allows the analysis of vibrational and/or rotational modes of a molecule.

The term "Raman spectroscopy" as used herein is defined as a spectroscopic technique used to study vibrational and/or rotational modes in a system, and relies on inelastic scattering (also referred to as Raman scattering) of monochromatic light, usually from a laser in the visible, near infrared, or near ultraviolet range. The incident laser light can lose or gain quanta of vibrational and/or rotational energy from the system, which results in a change of energy of the laser photons. This change in energy of the laser photons causes a spectral shift and provides information on the vibrational and/or rotational modes in the system. Typically, a sample is illuminated with a laser beam. Light from the illuminated spot is collected with a lens and sent through a spectrometer. Wavelengths close to the laser line (due to elastic Rayleigh scattering) are filtered out and those in a certain spectral window away from the laser line are dispersed onto a detector.

A Raman spectrum is a set of very narrow spectral lines emitted from object molecules when illuminated by an incident light. The width of each spectral line is strongly affected by the spectral width of the incident light and hence tightly monochromatic light sources, such as lasers, are used. The wavelength of each Raman line is expressed as a wavenumber-shift from the incident light, which is the difference between the wavenumber of the Raman line and the incident light. The wavenumber-shift, not the absolute wavenumber, of the Raman lines is specific to particular atomic groups in molecules. Raman spectra measure the vibrational and/or rotational modes of molecules which are determined by their molecular structure, especially by atomic groups such as methylene, ethylene, amide, phosphate or sulphide.

Most applications of Raman spectroscopy in biology are concerned with change in vibrational and/or rotational modes of macromolecules or related small molecules. Changes in either the wavenumber-shift of single Raman lines or the relative intensities of two or more Raman lines in an atomic group have been interpreted as indicating conformational changes in macromolecules. For these reasons Raman spectroscopy is mainly used for qualitative studies of molecules and molecular dynamics in biology. For easier and clearer interpretation of Raman spectra, use of the technique has been restricted mainly to purified materials and their systems, such as enzyme reactions. However, because Raman spectra are based on the specific vibrations and/or rotations of atomic groups they can also be used to characterise and quantify a mixture of molecules as compositions of atomic groups by a method akin to fingerprinting. Although unable to completely resolve the composition of a sample in terms of a list of chemical compounds, but for the most abundant molecular species, it does give a rough sketch of the molecular composition of the natural environment and how it changes with time.

The term "local natural moisturising factor content" as used herein is defined as the NMF content or NMF concentration in the stratum corneum at a given location on the body, such as the volar aspect of the forearm or the thenar (palm of the hand) and at a given distance below the skin surface.

The term "compound heterozygote" indicates the presence of two different mutant alleles at a particular gene locus, one on each chromosome of a pair. The genome contains two copies of each gene, a paternal and a maternal allele. A mutation affecting only one allele is called heterozygous. A "homozygous mutation" is the presence of the identical mutation on both alleles of a specific gene. However, when both alleles of a gene harbor mutations, but the mutations are different, these mutations are called compound heterozygous.

As has been illustrated in WO 2009/002149, it can be determined by measuring the NMF content in the stratum corneum whether a person has one or more mutations in the filaggrin gene. In this document, it has also been postulated that such a method can also distinguish between a heterozygous carrier of a filaggrin mutation and a homozygous carrier of the filaggrin mutation, which distinction is made solely on the basis of the concentration of NMF in said subject.

The current inventors have now found that the level of the amino acid tyrosine in the skin of individuals strongly correlated with the presence of two mutations in the filaggrin gene, whereas the absence of locations with elevated Tyr in the skin of individuals strongly correlates with the presence of one or zero mutations in the filaggrin gene. Thus, this finding enables a distinction between homozygous carriers on the one hand and heterozygous carriers or subjects having no mutation at all on the other hand. Thus, in conjunction with the method as disclosed in WO 2009/002149 it enables a distinction between subjects without mutation, subjects that are heterozygous carriers and subjects that are homozygous carriers.

Once it has been established that a subject contains a filaggrin mutation, such as by performing a method as disclosed in WO 2009/002149, the present invention provides a method to determine whether said subject is a homozygous or heterozygous carrier.

As said above, the concentration of the amino acid tyrosine in the skin is an indicator for the presence of one or two mutations in the filaggrin gene. Thus, in a subject in whom a filaggrin mutation has been detected, a next determination of the tyrosine concentration, or determination of the presence of regions with elevated Tyr content without actually determining the concentration of Tyr, would enable a classification whether such a mutation is heterozygous or homozygous.

In one embodiment the Tyr content in the skin is measured by a method of chemical analysis, for instance by chromatography. It should be understood that the Tyr concentration is preferably determined in the stratum corneum of the skin, and most preferably in the stratum corneum of the palm of the hand (thenar). However, the present method would equally be applicable to other places on or layers of the skin.

In a preferred embodiment, the Tyr content is determined from the intensity of the vibrational signal of Tyr, relative to the intensity of the vibrational signal of an internal reference. A suitable internal reference is for instance keratin.

In a preferred embodiment, the Tyr content is determined by spectral fitting. From a reference set of vibrational spectra that comprise the vibrational spectrum of skin or most of the vibrational spectrum of skin, the contribution of each reference spectrum to the vibrational spectrum of skin is determined. The relative contributions may be determined by fitting the reference spectra or selected spectral regions of the reference spectra to the vibrational spectrum of skin or selected spectral regions of the vibrational spectrum of skin. The fit coefficients represent the relative contributions of each of the reference spectra [Caspers et al. J. Invest. Dermatol. 2001, 116, 434-442]. Preferably, one of the reference spectra is a spectrum of Tyr. Preferably, one of the other reference spectra is a spectrum of NMF. Alternatively, one or more reference spectra are spectra of constituents of NMF. The set of reference spectra may be collected *in vitro* from pure skin constituents, solutions of pure skin constituents and/or assemblies of pure skin constituents. A particularly preferred skin constituent that may be used for a reference spectrum is keratin. Spectral contributions from Tyr can be commonly observed in Raman spectra of biological tissues, including spectra of the skin. Spectral contributions from Tyr are common for the Raman spectrum of keratin, which is the major constituent of the stratum corneum of the skin. When the spectral contribution from Tyr in a sample of the skin is stronger than the spectral contribution from Tyr in keratin, e.g. when the intensity of the spectral contribution from Tyr is above a previously set threshold, the sample is diagnosed as containing elevated Tyr.

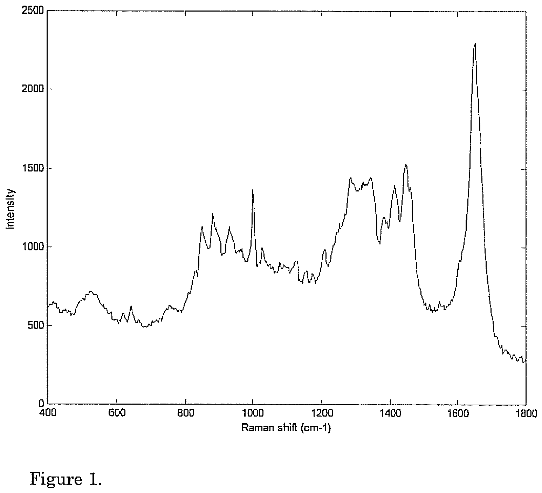

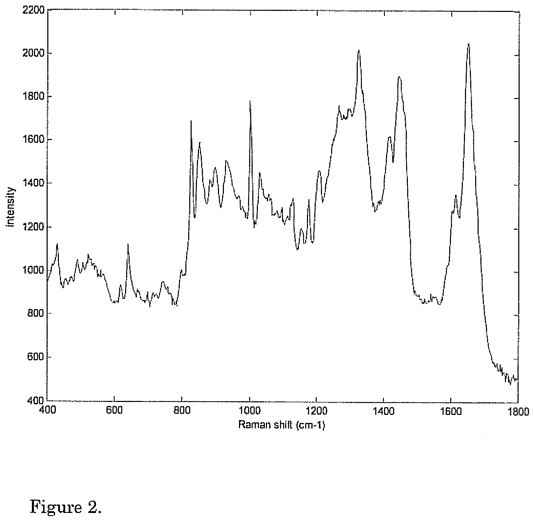

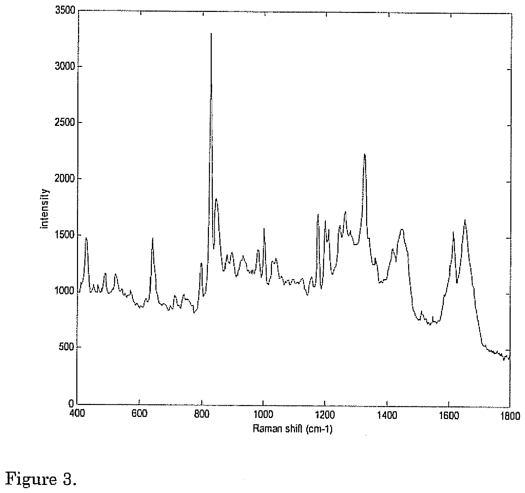

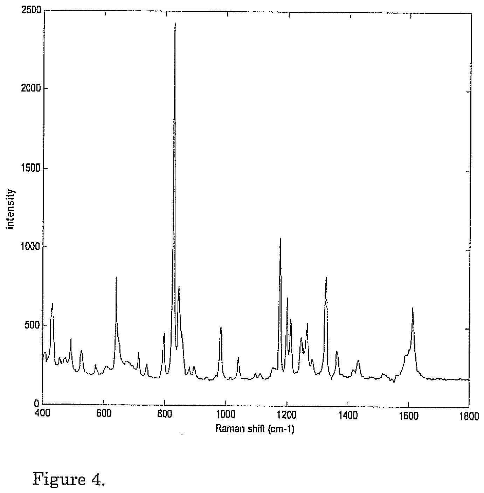

In another embodiment, the Tyr content is determined by calculation of the intensity of one or more peaks in the vibrational spectrum of Tyr, and calculation of the intensity of one or more peaks in the spectrum of an internal reference. A suitable internal reference is for instance keratin. However, also other common constituents of the stratum corneum can be used as suitable internal reference. This can be visualized in Figs. 1-4, wherein in Fig. 4 the Raman shift peak of (pure) Tyr is shown. Thus, in a spectrum from a sample it should be established whether also at this wavelength (at about 830 cm⁻¹) a peak is visible (see for example Figs. 2 and 3). In order to compensate for difference in the absolute value of the data, it is preferred to calculate the intensity of the Tyr peak with respect to an internal control (e.g. the value of the Raman spectrum at about 790 cm⁻¹). If this is more than a previous set threshold ratio (e.g. 1.2 or 1.3) the presence of a Tyr peak is established.

Vibrational spectra, and more in particular Raman spectra, can be analysed automatically and in real-time on a personal computer, which is programmed to calculate the Tyr content in the stratum corneum from a vibrational spectrum. This makes the result of the analysis instantly available. In another embodiment the vibrational spectra are stored and analysed at a later time.

Preferably, the Tyr content is determined by recording vibrational spectra *in vivo,* directly on the skin. However, the Tyr content can also be determined by recording vibrational spectra *ex vivo* on a stratum corneum sample that has been taken from the individual.

Preferably the Tyr content in the skin is determined by measuring vibrational spectra at a fixed and optimal distance from the skin surface at a given body location, preferably in the central part of the stratum corneum. The vibrational spectra of the stratum corneum on the thenar can be measured at a point 1-70 µm beneath the skin surface, more preferably at a point 2-50 µm beneath the skin surface.

In another embodiment the Tyr content can be measured at a number of different distances below the skin surface, for instance at three different distances below the skin. Preferably, when measuring on the thenar, the Tyr content is measured at about 30, 40 and 50 µm beneath the skin surface.

It has been found that the areas in which the Tyr content is high are localized spots, especially in the stratum corneum of the hand (thenar). It was found that, when employing a depth resolution of 5 µm, a spot with a high Tyr content could be observed at a given depth below the skin surface and no elevated Tyr would be detected at distances as small as 5 µm above or below that given point. This indicates that the localized spots with high Tyr content are confined to volumes that are well below the probing volume of the instrument. The estimated diameter of the Tyr spots is less than 5 µm, more likely less than 2.5 µm. The frequency of the Tyr spots was about 1 in every 4 measurements in a patient's skin with elevated Tyr content, which means that there is a near 100% incidence of encountering Tyr spots when measuring a patient's skin.

In a preferred embodiment the measuring volume of the Raman instrument is comparable to the volume of the regions of high Tyr content, for instance 10 µm³. This would require repeated measurements to determine the percentage of measurements in which a region with high Tyr is detected, typically between 20 and 50.

In another embodiment the measuring volume of the Raman instrument is larger than the volume of the regions of high Tyr content, for instance 100 µm³. In such a case fewer measurements are required. The chance of detecting a region with high Tyr per measurement then increases, but the contrast between measurements that include a region with high Tyr and measurements without a region of high Tyr is lower.

One or more vibrational spectra can be measured in the stratum corneum of an individual. It is preferred that more than one spectrum is measured, for instance at least 5 spectra, preferably at least 10 spectra, and more preferably at least 50 spectra. Measurements of the Tyr content may be repeated several times (such as 5-50 times) on slightly different locations, in order to increase the chance that a measurement coincides with a region of high Tyr. The slightly different locations can for instance be a translation over 0.05-1 mm, preferably 0.1-0.75 mm, more preferably 0.2-0.5 mm.

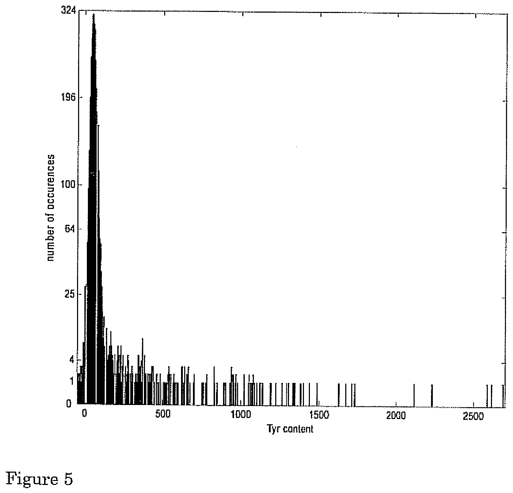

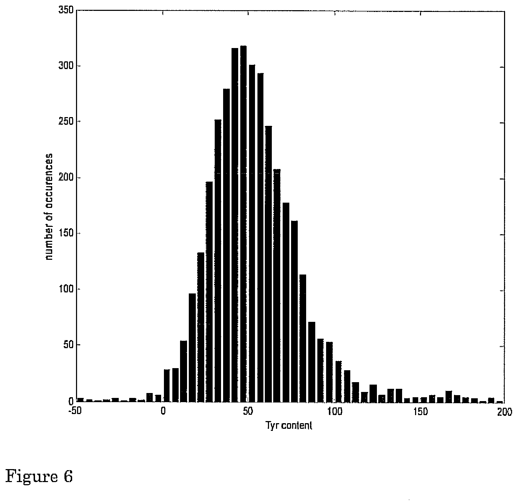

The Tyr content can be determined from the vibrational spectra. In one embodiment a subject can be defined Tyr-positive if the highest Tyr content in any of the repeated measurements is above a given threshold. An appropriate threshold can be set, based on a number of Raman spectra from skin with both normal and elevated Tyr content, and used thereafter. A threshold can be based on the average and the standard deviation in the Tyr content in normal skin. For example from figures 5 and 6 it is clear the Tyr content in the majority of the measurements approximates a normal distribution with an average value of 50, which can be called the normal Tyr content, and a standard deviation of 23. A threshold can now be set at a value equal to the average Tyr content plus 2 times the standard deviation, preferably to the average Tyr content plus 3 times the standard deviation, more preferably to the average Tyr content plus 4 times the standard deviation. In the example given below, the threshold for elevated Tyr is set at 150, which is the normal Tyr content plus 4 times the standard deviation. It will be clear to a person skilled in the art that the actual choice of the Tyr-threshold or the actual method by which a Tyr-threshold is determined may be varied, without materially deviating from the current invention.

If the Tyr content in all repeated measurements is below the given threshold, the subject can be defined Tyr-negative. A Tyr-positive subject is likely to have a homozygous or compound heterozygous mutation in the filaggrin gene and a Tyr-negative subject is not likely to have a homozygous or compound heterozygous mutation in the filaggrin gene.

In one embodiment the determination whether the highest Tyr content in the repeated measurements is above the given threshold, or whether the Tyr content in all repeated measurements is below the given threshold, can be combined with the measured NMF content according to WO 2009/002149, in order to determine whether a person is likely to have no, one or two filaggrin mutations. If the average measured NMF content has a value greater than a given threshold, for example 1.1, said person can be classified as having no filaggrin mutations. If the average measured NMF content is below a different threshold, for example 0.8, and if said person is Tyr-positive said person can be classified as having 2 filaggrin mutations. In all other cases said person can be classified as having a single filaggrin mutation.

In a specially preferred embodiment, the vibrational spectra are recorded on the thenar of the individual. Another preferred location to perform the vibrational spectra is the hypothenar eminence of the hand. Yet, in principle the vibrational spectra may be recorded on any other part of the body surface of the individual.

Preferably, the vibrational spectra are measured by Raman spectroscopy. In a special embodiment an in vivo confocal Raman microspectrometer as described by Caspers *et al.* may be used to record the vibrational spectra. [Caspers et al. Biospectroscopy 1998, 4, 31-39 and Caspers et al. J. Invest. Dermatol. 2001, 116, 434-442] Another example of an in vivo confocal Raman microspectrometer is the model 3510 Skin Composition Analyzer (River Diagnostics, Rotterdam, The Netherlands). However, also a simple Raman spectrometer is suitable for carrying out the method of the invention.

In a simple and cheap embodiment the laser light is focused at a fixed distance from the skin surface. In the case of the relatively thick stratum corneum of the thenar in the order of 100 µm thick, this fixed distance can be at 1-70 µm below the skin surface, preferably 2-60 µm, more preferably 3-50 µm. This eliminates the need for an accurate dynamic focussing device, which is part of commercially available confocal systems, such as the Model 3510 Skin Composition Analyzer (River Diagnostics). Such an accurate dynamic focussing device would needlessly drive up the cost of instruments to perform the methods according to the present invention.

It is not required that the Raman spectrometer has a high spatial resolution. In fact, a moderate or low spatial resolution can be of advantage, since it enables signal collection from *e.g.* a single relatively large part of the stratum corneum in one measurement. Thus, simpler and less expensive optical components in the light delivery and the light detection path can be used.

Further, a Raman spectrometer can be used, which detects several selected parts of the Raman spectrum with a low spectral resolution, and still provides sufficient information to distinguish between normal and aberrant Tyr content. The main signal contributions from Tyr occurs in the spectral windows 822-834 cm⁻¹ and 1320-1335 cm⁻¹, see Figure 4. In a preferred embodiment the Raman signal of Tyr is recorded in one or more of these spectral regions, and the Raman spectrum of the internal standard in a non- or partially overlapping spectral region.

A commercially available Raman skin analyser may be used. For example a Model 3510 Skin Composition Analyzer (River Diagnostics, Rotterdam, The netherlands) may be used. This system was designed for rapid, non-invasive analysis of the molecular composition of the skin. The device enables measurement of Raman spectra of the skin at a range of depths below the skin surface, and thereby enables quantitative and semi-quantitative analysis of molecular concentrations or contents in the skin as a function of distance to the skin surface.

The methods of the present invention can also be used to determine whether a specific individual is unsuitable, or at least less suitable, for a certain profession (such as hair-dresser or cook) or activity.

Further, on basis of the measurements and aligning the measurements with the type of atopic dermatosis of the subject, a classification can be made assigning dermatological characteristics to classes of NMF and/or Tyr content of the skin, or vice versa.

The method of the invention is an attractive and relatively cheap screening method. Based on the results from this screening it can be determined whether further (more expensive) screening or diagnosis is necessary.

With regards to therapy, the present invention can help to adjust the therapy by directing it more specifically to skin barrier impairment and can include administering of oral antihistamines, topical emollients, topical doxepin, topical corticosteroids, topical hydrocortisones, topical immunomodulators, and/or ultraviolet light therapy.

Further, it has appeared that such a method of determining the NMF and tyrosine content of the skin is also very advantageously applicable to find new mutations, especially in the filaggrin gene. To this end, Raman spectra of a patient are compared with Raman spectra of a subject that is known to have no mutation and with spectra from a subject that has a known mutation. If, in such a case, the Raman spectra of the patient show features that are similar (but not identical) to the spectra of the subject with the known mutation which are still different from such features from the spectra of the zero mutation control, it can be concluded that the patient would have a mutation that is different from the known mutation.

### Example

Raman spectra were recorded using the Model 3510 Skin Composition Analyzer (River Diagnostics BV, Rotterdam, The Netherlands). A reference Raman spectrum of solid L-tyrosine was measured with the sample placed directly on the measurement window of the Model 3510.

Raman spectra were recorded from 134 children with a well-described history of eczema and genotyped for the prevalent filaggrin mutations. For all patients Raman profiles were recorded *in vivo* on the palm of one hand in the mid-portion of the stratum corneum. A Raman profile consisted of 5 Raman spectra recorded from one spot on the skin at 30, 35, 40, 45 and 50 micron below the skin surface. The distance from the skin surface was determined as described in the User Manual of the Model 3510. Exposure time was 7 s per spectrum. For each patient on average 8 Raman profiles were recorded on slightly different spots on the thenar of the palm of the hand. This number was not always feasible, considering the young age of many patients, in which case less profiles were recorded. Prior to measurements the hand was gently wiped twice with a wet tissue in order to remove superficial dirt.

The wavenumber axis of the spectra was calibrated using the internal calibration standards of the Model 3510 Skin Composition Analyzer and instrument control software RiverICon (River Diagnostics). This software was also used to correct for the wavenumber dependent signal detection efficiency of the instrument. The NMF content was determined as described in WO 2009/002149 with the set of reference spectra expanded by a reference Raman spectrum of solid L-tyrosine. The Tyr content in a spectrum was calculated as the fit coefficient for L-tyrosine, divided by the fit coefficient for keratin, which was used as the internal reference. Spectra and fit results were exported to Matlab for further analysis. Single spectra containing one or more cosmic ray events, as well as single spectra with a signal-to-noise ratio (S/N) lower than 9 counts^{1/2} were excluded from the data set (S/N of a spectrum was defined as the S/N of the CH₂, calculated as the intensity of the 1448 cm⁻¹ band of keratin, divided by the square root of the total intensity at 1448 cm⁻¹).

The Raman spectra collected in this study revealed a surprisingly strong signal contribution from a substance in a sub-set of the patients and within those patients in a sub-set of the measurements. The substance was identified as the amino acid tyrosine (Tyr). In normal stratum corneum only Tyr incorporated in proteins - mainly keratin - contributes to the overall Raman spectrum of skin. Additional contributions from unbound Tyr are generally not detectable. In this study many spectra exhibited increased contributions of Tyr in the stratum corneum.

In nearly all homozygous and compound heterozygous individuals in the filaggrin gene, locations with significantly elevated Tyr concentration were detected.

In all subjects for which one ore more locations with elevated Tyr was detected, the detected NMF concentration was significantly lower than the average NMF concentration in subjects without a filaggrin mutation.

Classification of all patients as carrier of 0, 1 or 2 filaggrin mutations was performed and compared to genotyping as golden standard. When only the NMF content was used for classification, patients with an NMF content greater than 1.1 could be classified as carrier of no filaggrin mutations, patients with an NMF content lower than 0.8 could be classified as carrier of two mutations, and all other patients could be classified as carrier of one mutation. This resulted in 22 misclassifications for a total of 110 patients (42 patients genotyped with 0 mutations, 42 patients genotyped with 1 mutation and 26 patients genotyped with 2 mutations). Most misclassifications were carriers of 1 mutation, who were classified as carrier of 2 mutations. When both NMF content and elevated tyrosine were used for classification, patients with an NMF content greater than 1.1 could be classified as carrier of no filaggrin mutations, patients with an NMF content lower than 0.8 and elevated tyrosine could be classified as carrier of two mutations, and all other patients could be classified as carrier of one mutation. With this classification method the number of misclassifications decreased to 15. In particular carriers of 1 mutation and carriers of 2 mutations could be much better distinguished.

Analysis of the Raman spectra, based on the methods described above, of 7 patients with no filaggrin mutations according to the original genotyping showed features that were different from other patients without filaggrin mutations. For example in some patients the NMF content was surprisingly low, and in some patients locations with elevated Tyr were found. These patients were then screened more extensively for possible filaggrin mutations, which resulted in the discovery of additional mutations in 3 of the patients, and a previously undiagnosed case of X-linked ichtyosis in one of the patients. Similarly, analysis of the Raman spectra of 9 patients with a single filaggrin mutation (heterozygous) according to the original genotyping, showed features that were different from other patients with a single filaggrin mutation. These patients were then screened more extensively for possible filaggrin mutations, which resulted in the discovery of additional mutations in 4 of the patients. From the total of 8 mutations that were discovered after reassessment of the genotypes, 5 mutations were previously unknown mutations in the filaggrin gene.

The measurements in the example given here were performed with an axial spatial (depth) resolution of 5 µm and a measuring volume that is comparable to the volume of the localizations with high Tyr content. It is also possible to use a measuring volume of the Raman instrument that is larger than the volume of the regions of high Tyr content, for instance 100 µm³. Such measurements were simulated as follows. For each patient with at least one elevated Tyr measurement, a random selection of 10 Raman spectra were co-added, simulating a 10-fold larger measurement volume. For this summed spectrum was determined whether the Tyr content was above or below the given threshold, using the procedure described above. This was repeated 50 times, simulating for each patient a total of 50 measurements, each with a 10-fold larger measurement volume. The percentage of Tyr-positive determinations in the simulated measurements was then compared to the percentage of Tyr-positive measurements in the original data. As a result, about 50% of the simulated measurements with large measuring volume showed elevated Tyr, compared to about 25% of the original measurements, which is a 2-fold increase.

## Claims

1. A method to determine if a person has a homozygous or compound heterozygous mutation in the filaggrin-gene FLG comprising measurement of the presence of tyrosine in the skin.

2. A method according to claim 1 comprising the use of Raman spectroscopy to obtain a measure of tyrosine presence in the skin

3. A method to according to claim 2 comprising the use of Raman spectroscopy to obtain a measure of tyrosine presence in the *stratum corneum* of the skin

4. A method according to claim 3 comprising taking the Raman measurements of the palm of the hand.

5. A method according to claims 2-4 in which multiple Raman measurements are carried out at more than one location of the body.

6. A method according to claim 5 in which Raman measurements are carried out at more than one location of the hand.

7. A method according to any of claims 1-6 to determine if a person has a mutation in both FLG alleles, comprising measuring Raman spectra at different locations in the stratum corneum, determining if the tyrosine content is above a set threshold, whereby said person is likely to have a mutation in both FLG alleles or determining if the tyrosine content is below a set threshold, whereby said person is not likely to have a mutation in both FLG alleles.

8. A method to determine if a person has zero, one or two mutant FLG-genes comprising measurement of the presence of tyrosine and the concentration of natural moisturising factor NMF in the skin.

9. A method according to claim 8, comprising taking Raman spectra measurements at different locations in the stratum corneum, determining the NMF concentration and the presence tyrosine from the measured Raman spectra, determining that if the average NMF concentration is above a set threshold the said person is likely to have no FLG mutation, and determining that if the average NMF concentration is below a set threshold and the tyrosine content in all measurements is below a set threshold said person is likely to have a mutation in one FLG allele, and determining that if the average NMF concentration is below a set threshold and the tyrosine content in one or more measurements is above a set threshold said person is likely to have a mutation in both FLG alleles.

10. A method according to any of claims 1-9, wherein the measurement of tyrosine is performed *in vitro.*

11. A method according to any of the preceding claims to test subjects for suitability of inclusion in a test of a topically applied product.

12. A method according to any one of claims 1-10, for determining whether an individual is unsuitable, or at least less suitable, for a certain profession or activity.

13. A method to classify atopic dermatitis based on the NMF and tyrosine content in the skin of a subject.

## Patentansprüche

1. Verfahren zum Bestimmen, ob eine Person eine homozygote oder zusammengesetzte-heterozygote Mutation im Filaggrin-Gen FLG hat, umfassend Messung der Anwesenheit von Tyrosin in der Haut.

2. Verfahren nach Anspruch 1, umfassend die Verwendung von Raman-Spektroskopie, um ein Maß für Tyrosin Anwesenheit in der Haut zu erhalten.

3. Verfahren nach Anspruch 2, umfassend die Verwendung von Raman-Spektroskopie, um ein Maß für Tyrosin Anwesenheit in dem *stratum corneum* der Haut zu erhalten.

4. Verfahren nach Anspruch 3, umfassend Aufnehmen der Raman-Messungen der Handfläche.

5. Verfahren nach den Ansprüchen 2-4, in dem mehrere Raman-Messungen an mehr als einer Stelle des Körpers durchgeführt werden.

6. Verfahren nach Anspruch 5, in dem Raman-Messungen an mehr als einer Stelle der Hand durchgeführt werden.

7. Verfahren nach einem der Ansprüche 1- 6, zum Bestimmen, ob eine Person eine Mutation in beiden FLG-Allelen hat, umfassend Messen von Raman-Spektren an verschiedenen Stellen im stratum corneum, Bestimmen, ob der Tyrosingehalt über einer festgelegten Schwelle ist, wonach die Person wahrscheinlich eine Mutation in beiden FLG-Allelen hat, oder Bestimmen, ob der Tyrosingehalt unter einer festgelegten Schwelle ist, wonach die Person wahrscheinlich nicht in beiden FLG-Allelen eine Mutation hat.

8. Verfahren zur Bestimmen, ob eine Person null, ein oder zwei Mutanten-FLG-Gene hat, umfassend Messung der Anwesenheit von Tyrosin und der Konzentration des natürlichen Feuchtigkeitsfaktors NMF in der Haut.

9. Verfahren nach Anspruch 8, umfassend Aufnehmen von Raman-Spektren Messungen an verschiedenen Stellen im stratum corneum, Bestimmender NMF-Konzentration und der Anwesenheit von Tyrosin aus den gemessenen Raman-Spektren, Bestimmen, dass, wenn die durchschnittliche NMF-Konzentration über einer festgelegten Schwelle ist, die Person wahrscheinlich keine FLG-Mutation hat, und Bestimmen, dass, wenn die durchschnittliche NMF-Konzentration unter einer festgelegten Schwelle ist und der Tyrosingehalt in allen Messungen unter einer festgelegten Schwelle ist, die Person wahrscheinlich eine Mutation in einem FLG-Allel hat, und Bestimmen, dass, wenn die durchschnittliche NMF-Konzentration unter einer festgelegten Schwelle ist und der Tyrosingehalt in einer oder mehreren Messungen über einer festgelegten Schwelle ist, die Person wahrscheinlich eine Mutation in beiden FLG-Allelen hat.

10. Verfahren nach einem der Ansprüche 1-9, wobei die Messung von Tyrosin *in vitro* durchgeführt wird.

11. Verfahren nach einem der vorhergehenden Ansprüche, um Subjekte auf Eignung für die Aufnahme in einen Test eines topisch applizierten Produkts zu testen.

12. Verfahren nach einem der Ansprüche 1-10 zum Bestimmen, ob ein Individuum für einen bestimmten Beruf oder Tätigkeit ungeeignet oder mindestens weniger geeignet ist.

13. Verfahren, um atopische Dermatitis basierend auf dem NMF- und Tyrosingehalt in der Haut eines Subjekts zu klassifizieren.

## Revendications

1. Procédé pour déterminer si une personne a une mutation homozygote ou hétérozygote composite dans le gène de la filaggrine FLG comprenant une mesure de la présence de tyrosine dans la peau.

2. Procédé selon la revendication 1 comprenant l'utilisation de la spectroscopie Raman pour obtenir une mesure de la présence de tyrosine dans la peau.

3. Procédé selon la revendication 2 comprenant l'utilisation de la spectroscopie Raman pour obtenir une mesure de la présence de tyrosine dans la couche cornée de la peau.

4. Procédé selon la revendication 3 comprenant la prise des mesures Raman de la paume de la main.

5. Procédé selon les revendications 2-4 dans lequel de multiples mesures Raman sont faites à plus d'un emplacement du corps.

6. Procédé selon la revendication 5 dans lequel des mesures Raman sont faites à plus d'un emplacement de la main.

7. Procédé selon l'une quelconque des revendications 1-6 pour déterminer si une personne a une mutation dans les deux allèles du FLG, comprenant le fait de mesurer des spectres Raman à différents emplacements dans la couche cornée, de déterminer si la teneur en tyrosine est au-dessus d'un seuil fixé, de sorte que ladite personne est susceptible d'avoir une mutation dans les deux gènes du FLG ou de déterminer si la teneur en tyrosine est en-dessous d'un seuil fixé, de sorte que ladite personne n'est pas susceptible d'avoir une mutation dans les deux allèles du FLG.

8. Procédé pour déterminer si une personne a zéro, un ou deux gènes FLG mutants comprenant la mesure de la présence de tyrosine et de la concentration de facteur naturel d'hydratation FNH dans la peau.

9. Procédé selon la revendication 8, comprenant la prise de mesures de spectres Raman à différents emplacements dans la couche cornée, la détermination de la concentration de FNH et de la présence de tyrosine d'après les spectres Raman mesurés, la détermination de ce que si la concentration de FNH moyenne est au-dessus d'un seuil fixé ladite personne est susceptible de n'avoir aucune mutation du FLG, et la détermination de ce que si la concentration de FNH moyenne est en-dessous d'un seuil fixé et la teneur en tyrosine dans toutes les mesures est en-dessous d'un seuil fixé ladite personne est susceptible d'avoir une mutation dans un allèle du FLG, et la détermination de ce que si la concentration de FNH moyenne est en-dessous d'un seuil fixé et la teneur en tyrosine dans une ou plusieurs mesures est au-dessus d'un seuil fixé ladite personne est susceptible d'avoir une mutation dans les deux allèles du FLG.

10. Procédé selon l'une quelconque des revendications 1-9, dans lequel la mesure de la tyrosine est réalisée *in vitro.*

11. Procédé selon l'une quelconque des revendications précédentes pour tester des sujets pour le caractère approprié de l'inclusion dans un test d'un produit appliqué de manière topique.

12. Procédé selon l'une quelconque des revendications 1-10, pour déterminer si un individu n'est pas approprié, ou au moins moins approprié, pour une certaine profession ou activité.

13. Procédé pour la classification de la dermatite atopique sur la base de la teneur en FNH et en tyrosine dans la peau d'un sujet.
